# EUROPEAN PATENT APPLICATION

(11) **EP 2 543 328 A1**
(43) Date of publication of application: **09.01.2013**
(21) Application number: 12006811.9
(22) Date of filing: 20.11.2007
(51) Int. Cl.: A61B 17/17, A61B 17/80

(54) **Variable angle drill guide**

(30) Priority: 21.11.2006 US 866665 P
(62) Divisional of application: 07864648.6
(71) Applicant: SMITH & NEPHEW, INC., Memphis, TN 38116 (US)
(72) Inventor: Chreene, David E., Hernando Mississippi 38632 (US); Baker, Charles R, Lakeland TN 38002 (US); Stewart, William W, Memphis TN 38104 (US); Zahlry, Daniel C, Germantown TN 38139 (US)
(74) Representative: Guy, Mark Robert

(57) **Abstract**

There is provided a drill guide assembly (10) adapted for removable connection to a bone plate (200, 310, 1150, 1250) and for guiding a drill bit (300) through at least one hole (202, 312) that has a central axis, The drill guide assembly (10) includes a guide sleeve (80, 600, 910, 952, 1000, 1052, 1110, 1210) and a guide collar (30) connected to the guide sleeve. The guide collar has an outrigger (40, 500, 800), and the outrigger has at least one opening (42, 504, 804) adapted to receive the drill bit. The at least one opening allows an operator to select an angle for placement of the drill bit and limits the maximum angle selectable by the operator.

## Description

### CROSS-REFERENCE TO RELATED APPLICATIONS

This application claims the benefit of U.S. Provisional Application No. 60/866,665, filed 21 November 2006. The disclosure of this prior application is incorporated by reference in its entirety.

### BACKGROUND OF THE INVENTION

### FIELD OF THE INVENTION

The present invention relates generally to surgical instruments and, more particularly, to a variable angle drill guide.

### RELATED ART

Presently, variable angle drill guides for use with bone plating in the medical field require the user to drill through multiple sleeves or require multiple complex components of assembly to pivot for off-axis drilling. It would be desirable to provide the ability to rotate 360 degrees relative to the longitudinal axis of the drill guide while drilling on or off-axis. It also would be desirable to angle the drill bit through a single sleeve or guide feature while the drill guide itself is oriented transverse to the bone plate surface, which in turn ensures that a fastener is located in the center of the hole or other plate opening while drilling at any angle.

International Patent Application No. PCT/US2005/012116 discloses a surgical drill guide for use with a bone plate having fastener holes oriented at predetermined angles with respect to the plate. The surgical drill guide has at least one alignment drill guide barrel that is aligned with the respective fastener holes in the bone plate for drilling holes at the desired range of angles permitted by the plate hole. However, this reference does not disclose a mechanism that positively engages the bone plate nor does it provide a limit on the angularity of the drill bit.

International Patent Application No. PCT/CH01/00221 discloses a surgical drill guide for demountable attachment to a slotted bone plate. The drill guide assembly includes one or more alignment drill tubes that are remotely aligned with corresponding fastener holes in the bone plate and an expandable bushing that is configured and dimensioned to engage a slot in the bone plate. A variable angle block permits angulation of the alignment drill tubes about a central axis of the surgical drill guide assembly. The surgical drill tubes are releasably lockable at a surgeon-selected angle. However, this reference fails to disclose a drill guide proximate to the bone plate.

There remains a need in the art for a variable angle drill guide that provides support for the drill bit proximate a bone plate and limits the maximum angle of the drill bit relative to the bone plate hole.

### SUMMARY OF THE INVENTION

There is provided a drill guide assembly adapted for removable connection to a bone plate and for guiding a drill bit through at least one hole of the bone plate, the at least one hole having a central axis, the drill guide assembly comprising: a guide sleeve having a proximal end portion and a distal end portion, the guide sleeve coaxial with the central axis and adapted to swivel about the central axis while the distal end portion maintains contact with the bone plate; and a guide collar operatively connected to the proximal end portion, the guide collar having an outrigger, and the outrigger having at least one opening adapted to receive the drill bit, and the at least one opening allowing an operator to select an angle for placement of the drill bit and adapted to limit the maximum angle selectable by the operator.

In some embodiments, the outrigger further comprises a flanged bobbin.

In some embodiments, the outrigger further comprises a plurality of scallops.

In some embodiments, the drill guide assembly further comprises at least one ball plunger.

In some embodiments, the guide collar includes a base and a body portion, and the outrigger extends generally from the body portion.

In some embodiments, the guide sleeve includes a sleeve body and a passage.

In some embodiments, the guide sleeve further comprises at least one marker groove,

In some embodiments, the outrigger includes one or more markings to indicate an angle of the drill bit.

In some embodiments, the drill guide assembly further comprises a drill guide handle, the drill guide handle having a grip portion and an engagement portion.

In some embodiments, the drill guide handle further comprises a pivot portion.

In some embodiments, the distal end portion of the guide sleeve comprises a spherical tip.

In some embodiments, the drill guide assembly further comprises a bushing and a distal guide tip.

In some embodiments, the drill guide assembly further comprises a limiter. The limiter may or may not threadingly engage the guide sleeve.

In some embodiments, the distal end portion of the guide sleeve comprises a drill guide tip. The drill guide tip may include an anti-rotation feature. The anti-rotation feature may be a locating boss, a locating member, or comprise at least one thread adapted to mate with at least one thread of the at least one bone plate hole.

In some embodiment, the guide sleeve further comprises at least one tang and the drill guide tip further comprises an under-cut groove adapted to receive the at least one tang.

In some embodiments, the drill guide tip further comprises a cone.

There is also provided a method of drilling a hole into bone through a bone plate, the method comprising: providing a variable angle drill guide assembly; engaging a drill guide tip of the variable angle drill guide assembly in contact with the bone plate; selecting a rotational position; inserting a drill bit into the variable angle drill guide assembly; selecting an angular position of the drill bit; and drilling the bone.

There is further provided a drill guide assembly adapted for removable connection to a bone plate and for guiding a drill bit through at least one hole of the bone plate, the at least one hole having a central axis, the drill guide assembly comprising: a guide sleeve assembly having a proximal end portion and a distal end portion, the guide sleeve assembly coaxial with the central axis and adapted to swivel about the central axis while the distal end portion maintains contact with the bone plate, the guide sleeve assembly comprising an outer guide sleeve and an inner guide sleeve pivotally connected to the outer guide sleeve, the inner guide sleeve having at least one opening adapted to receive the drill bit, and the at least one opening allowing an operator to select an angle for placement of the drill bit and adapted to limit the maximum angle selectable by the operator.

There is also provided a drill guide assembly for guiding a drill bit through at least one hole of a bone plate, the drill guide assembly comprising: a guide collar, the guide collar having an outrigger, and the outrigger having a slot adapted to receive the drill bit; a guide sleeve connected to the guide collar, the guide sleeve having a passage and a length, the passage extending at least a portion of the length of the guide sleeve; and a drill guide tip connected to the guide sleeve, the drill guide tip adapted for removable connection to the bone plate, and the drill guide tip having a cone, wherein a movement of the drill is limited by the slot, the passage, and the cone.

There is also provided a drill guide assembly for guiding a drill bit through at least one hole of a bone plate, the drill guide assembly comprising: a guide collar, the guide collar having a base, a body portion, and an outrigger, the body portion extending from the base in a transverse direction, the outrigger extending from body portion in a generally arcuate direction, and the outrigger having a slot adapted to receive the drill bit and limit the maximum angle of the drill bit; a guide sleeve connected to the guide collar, the guide sleeve having a sleeve body, a bore, a passage, a plurality of tangs, and a length, the bore extending the length of the sleeve body, and the passage extending at least a portion of the length; and a drill guide tip connected to the guide sleeve, the drill guide tip having an under-cut groove to receive the plurality of tangs, a cone, and a locating boss, the locating boss adapted for removable connection to the bone plate, and wherein a movement of the drill is limited by the slot, the passage, and the cone.

There is provided a variable angle drill guide assembly for use in bone plating applications. The variable angle drill guide assembly includes a guide collar, a guide sleeve, and a guide tip. The guide tip interfaces with a bone plate in a transverse manner and positions the variable angle drill guide in the center of a bone plate hole. The variable angle drill guide is applicable for use with either locking or non-locking holes. The variable angle drill guide allows the user to drill on or off-axis at any angle from about zero to about thirty degrees, as well as in any direction of 360 degrees of rotation about the longitudinal axis of the drill guide.

The variable angle drill guide assembly offers more opportunities for fracture fixation in comparison to prior devices. The rotational freedom and angular freedom increases the user's opportunities for bone fracture fixation. In particular, the rotational freedom is not limited to predetermined hole locations of the bone plate.

The variable angle drill guide assembly provides an advantage over prior devices because it is simpler to use and reduces the overall number of instruments required for the surgical technique. The variable angle drill guide assembly eliminates the need for multiple barrels or guide sleeves, which also reduces the time required to complete the surgical technique.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The accompanying drawings, which are incorporated in and form a part of the specification, illustrate the embodiments of the present invention and together with the written description serve to explain the principles, characteristics, and features of the invention. In the drawings:

FIG. 1 is an exploded, front perspective view of a variable angle drill guide assembly, handle and bone plate;

FIG. 2 is an exploded, front perspective view of a guide collar;

FIG. 3 is a front perspective view of a guide sleeve;

FIG. 4 is a partial sectional front view of a guide tip;

FIG. 5 is a bottom view of the guide tip in a first embodiment;

FIG. 6 is a bottom view of the guide tip in a second embodiment;

FIG. 7 is a bottom view of the guide tip in a third embodiment;

FIG. 8 is a front perspective view of the variable angle drill guide assembly, a bone, and a bone plate;

FIG. 9 is a sectional front view of the variable angle drill guide assembly shown in FIG. 8;

FIG. 10 is a front perspective view of an alternative embodiment of the handle;

FIG.11 is a sectional side view of the handle shown in FIG. 10;

FIG. 12 is a sectional side view of the outrigger in a second embodiment;

FIG. 13 is a sectional side view of the guide sleeve in a second embodiment;

FIG. 14 is a sectional side view of the guide sleeve in a third embodiment;

FIG. 15 is a partial top view of the outrigger in a third embodiment;

FIG. 16 is a sectional side view of the guide sleeve in a fourth embodiment;

FIG. 17 is a sectional side view of the guide sleeve in a fifth embodiment;

FIG. 18 is a sectional side view of the guide sleeve in a sixth embodiment;

FIG. 19 is a sectional side view of the guide sleeve in a seventh embodiment;

FIG. 20 is a front perspective view of an alternative embodiment of the drill guide assembly; and

FIG. 21 is a front view of an alternative method of limiting the relationship between the bone plate and the drill guide assembly.

### DETAILED DESCRIPTION OF THE EMBODIMENTS

The following description of the preferred embodiment(s) is merely exemplary in nature and is in no way intended to limit the invention, its application, or uses.

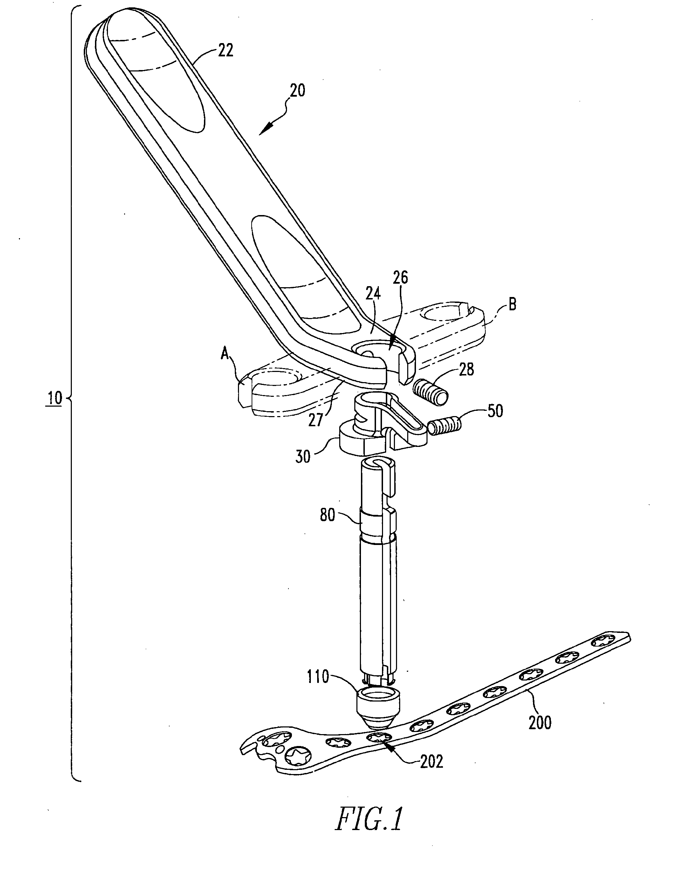

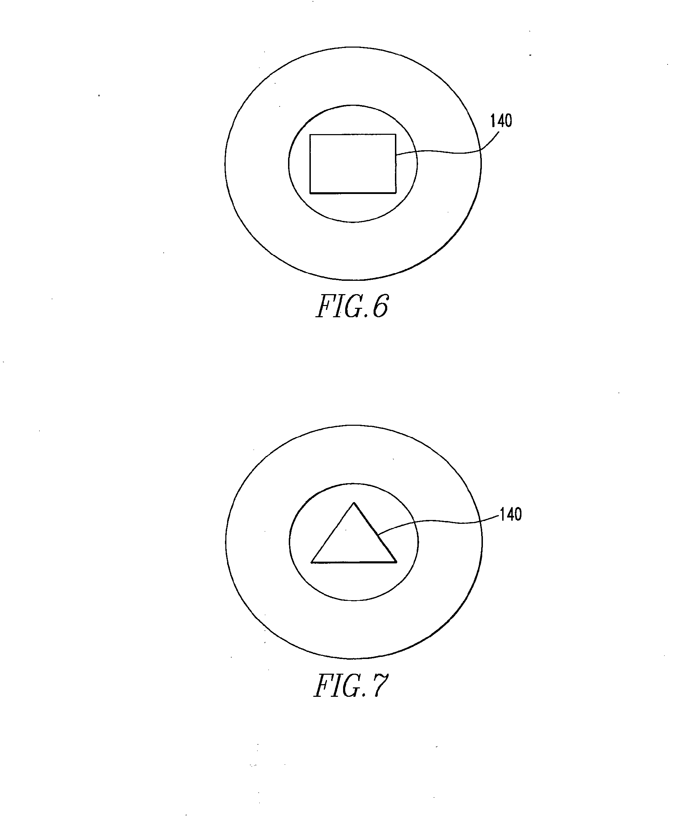

FIG. 1 is an exploded view of a drill guide assembly 10. In the depicted embodiment, the drill guide assembly 10 includes a guide collar 30, a guide sleeve 80, and a drill guide tip 110. The drill guide assembly 10 may include a drill guide handle 20. The drill guide handle 20 may be removably attached to a portion of the drill guide assembly 10. The drill guide assembly 10 is adapted for removable connection to a bone plate 200 and for guiding a drill bit 300 (best seen in FIG. 6) through a hole 202 in the bone plate 200. As is explained in greater detail below, after the drill guide handle 20 connects to a portion of the drill guide assembly 10 and the drill guide assembly 10 is resting on the bone plate 200, the drill guide handle 20 may be used to pivot the guide sleeve 80 to obtain a desired location of the drill bit 300. Phantom lines A and B illustrate exemplary rotational positions of the handle 20 but the handle 20 may be pivoted to other rotational positions as well.

The drill guide assembly 10 may be made from a metal, plastic, or composite. In the depicted embodiments, the drill guide assembly 10 is constructed from stainless steel and each piece of the assembly is a machined component. Alternatively, the drill guide assembly 10 could be an injection molded one-piece design.

The handle 20 includes a grip portion 22 and an engagement portion 24. In the depicted embodiment, grip portion 22 is transverse to the engagement portion 24. However, in some embodiments, the grip portion 22 is substantially planar with engagement portion 24. The engagement portion 24 includes an opening 26 and may have a generally planar lower surface 27. The opening 26 receives a portion of the guide collar 30, and, in some embodiments, the lower planar surface 27 engages the collar 30. In the depicted embodiment, the opening 26 is generally c-shaped. Optionally, the handle 20 includes a first ball plunger 28. The handle 20 may be made from any number of materials, including wood, metal, plastic, or a composite.

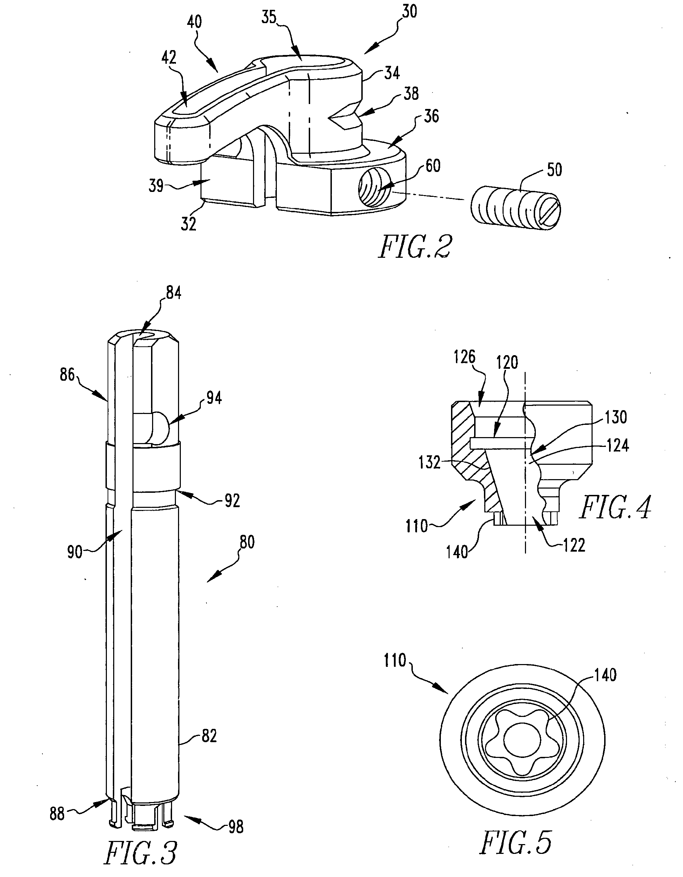

Referring now to FIG. 2, the guide collar 30 includes a base 32, a body portion 34, and an outrigger 40. The "outrigger" may alternatively be termed a "snout" or an "elongated feature." The body portion 34 extends from the base 32 in a transverse direction. In the depicted embodiment, the base 32 and the body portion 34 are generally cylindrical but other shapes may be used. The base 32 and the body portion 34 include a first bore 35. The body portion 34 is sized and dimensioned such that the base 32 provides a ledge 36. The ledge 36 provides a support for the handle 20. In some embodiments, the body portion 34 includes a first groove 38. The first groove 38 may be used in conjunction with the first ball plunger 28. In this manner, the handle 20 may be temporarily affixed to the collar 30. Optionally, the base 32 may includes a substantially planar face 39. In the depicted embodiment, the outrigger 40 extends in an arcuate direction from the body portion 34, but the outrigger 40 may extend transversely from the body portion 34 in other embodiments.

The outrigger 40 includes an opening, a hole, or a slot 42. In some embodiments, the slot 42 is dimensioned for a single drill size. For example, the slot 42 may be dimensioned to receive a 2.7 millimeter drill bit. In other embodiments, the slot 42 is dimensioned to receive multiple sizes of drill bits. The slot 42 connects to the first bore 35. The slot 42 guides the drill bit 300. The length of the slot 42 is dimensioned according to the desired maximum angle of the drill. The maximum angle may be as much as thirty degrees, but in the depicted embodiment the maximum angle is fifteen degrees. In some embodiments, the outrigger 40 may include markings indicating an angle of the drill relative to the central axis of the guide sleeve 80. For example, these markings may include text or other markers indicating 2.5 degrees, 5.0 degrees, 7.5 degrees, 10 degrees, 12.5 degrees, and 15 degrees. Optionally, the guide collar 30 may include a second ball plunger 50 and a threaded hole 60 to receive the second ball plunger 50. The second ball plunger 50 may be located on either side of the guide collar 30.

Referring now to FIG. 3, the guide sleeve 80 includes sleeve body 82, a second bore 84, a proximal end portion 86, a distal end portion 88, a passage 90, and one or more tangs 98. A length of the guide sleeve 80 is determined by a length of the drill bit 300. In other words, the length of the guide sleeve 80 is designed according to the length of the drill bit 300. The sleeve body may be any shape, but the sleeve body 82 is generally cylindrical in FIG. 3. In the depicted embodiment, the second bore 84 is generally coaxial with the sleeve body 82. The second bore 84 extends substantially the entire length of the sleeve body 82 and is dimensioned to receive the drill bit 300. The proximal end portion 86 is dimensioned to fit within the first bore 35 of the collar 30. The tangs 98 extend generally from the distal end portion 88. In the depicted embodiment, there are four tangs, but those of ordinary skill in the art would understand that a greater or lesser number of tangs may be used. Optionally, the guide sleeve 80 may include a marker groove 92. The marker groove 92 may be painted or colored to indicate the drill guide assembly 10 is designed for use with a particular size or shape of drill. Alternatively, the marker groove 92 may be painted or colored to indicate the drill guide assembly 10 is part of a particular kit. In some embodiments, the guide sleeve 80 may include a u-shaped cutout 94. The u-shaped cutout 94 provides clearance for the second ball plunger 50.

Referring to FIGS. 4 and 5, the drill guide tip 110 includes an under-cut groove 120, a distal opening 122, a central axis 124, a proximal opening 126, a cone 130, and a locating boss 140. The under-cut groove 120 receives the tangs 98. The distal opening 122 opens up into the hole 202 when the drill guide tip 110 is engaged with the bone plate 240. The proximal opening 126 is sized and dimensioned to receive the distal end portion 88 of the guide sleeve 80. The cone 130 guides the drill bit 300. In other words, the cone 130 limits the drill bit 300 as the drill moves in the slot 42. The cone 130 has a cone wall 132 that is angled relative to the central axis 124. The maximum angle may be as much as thirty degrees, but in the depicted embodiment the maximum angle is fifteen degrees. In the depicted embodiments, the maximum angle of the cone wall 132 coincides with the maximum angle provided by the slot 42.

The locating boss 140 is adapted to engage the hole 202 of the bone plate 200. The locating boss 140 may be threaded such that it may thread into the hole 202. Alternatively, the locating boss 140 may have a geometric shape that corresponds with a portion of the hole 202. As examples, the locating boss 140 may have a star shape (best seen in FIG. 5), a square or rectangular shape (best seen in FIG. 6), or a triangular shape (best seen in FIG. 7). However, those having ordinary skill in the art would understand that other shapes may be used. What is significant is that the locating boss 140 engages the hole 202 of the bone plate 200 and prevents the drill guide tip 110 from rotating.

The drill guide assembly 10 is assembled in the following steps. First, the collar 30 is assembled to the proximal end portion 86 of the guide sleeve 80. As noted above, the proximal end portion 86 is dimensioned to fit within the first bore 35 of the collar 30. The fit between the two the components may be a slip fit or a press fit. In some embodiments, a structural adhesive is applied to the proximal end portion 86 before it is assembled to the collar 30. The guide sleeve 80 is inserted into the collar 30 such that the passage 90 is aligned with the slot 42. Optionally, once the collar 30 is attached to the guide sleeve 80, the ball plunger 50 may be attached to the collar such that the ball protrudes into the passage 90. In this manner, the second ball plunger 50 acts as indicator to let the user know whether the drill bit 300 is on-axis or off axis. In other words, the second ball plunger 50 acts as indicator to let the user know whether or not the drill bit 300 is aligned with the second bore 84. Second, the drill guide tip 110 is pressed onto the distal end portion 88 of the guide sleeve 80 until the tangs 98 engage the under-cut groove 120. Once the tangs 98 engage and capture the under-cut groove 120, the drill guide tip 110 can rotate 360 degrees about the longitudinal axis of the guide sleeve 80 but cannot easily be removed from the guide sleeve. Third, the handle 20 connects to the collar 30. The opening 26 is placed over the body portion 34, and the engagement portion 24 is pressed downwardly until it engages the ledge 36. Optionally, the handle 20 may include the first ball plunger 28, and the first ball plunger 28 may engage the first groove 38. Thereafter, the handle 20 may be used to pick up the drill guide assembly 10 and place it relative to the bone plate 200.

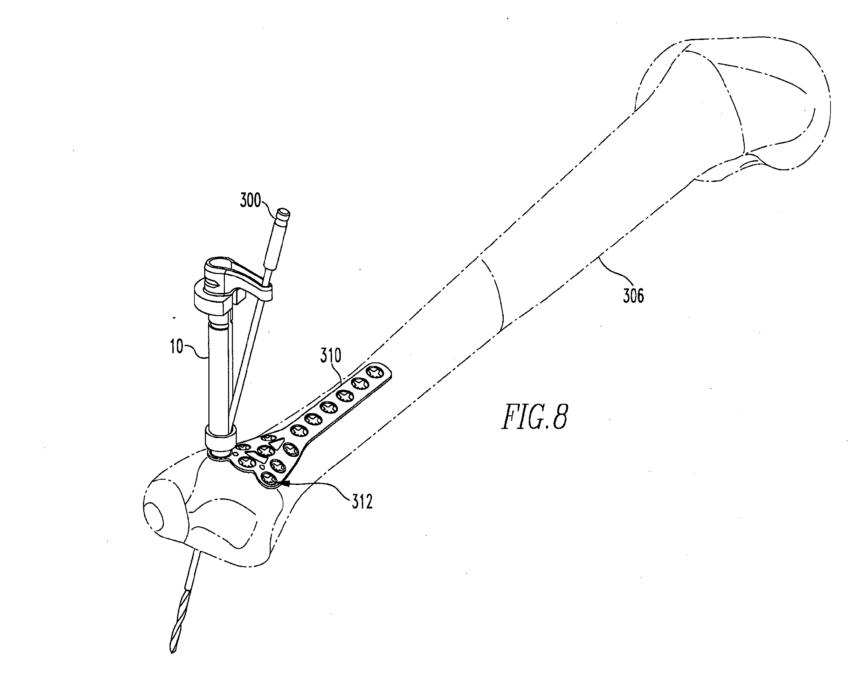

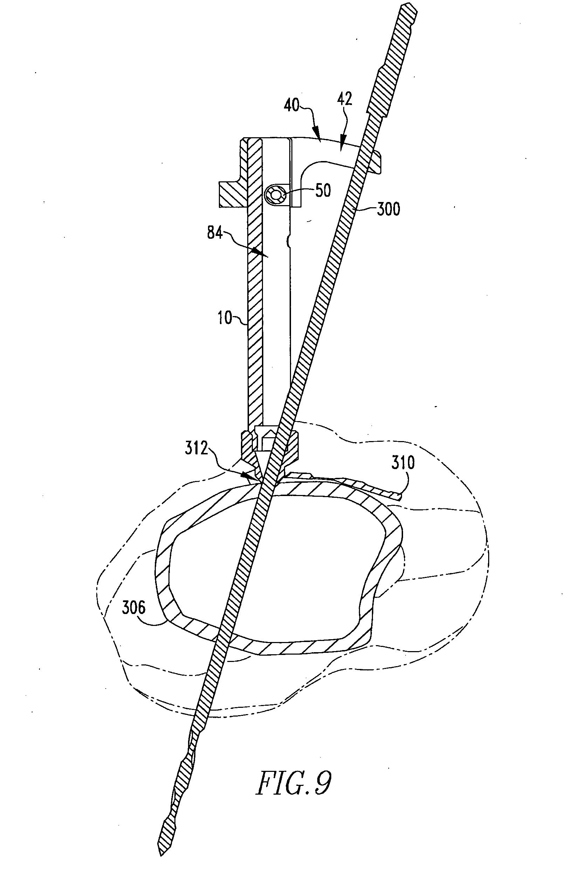

FIGS. 8 and 9 illustrate the drill guide assembly 10, the drill bit 300, a bone 306, and a bone plate 310. In the depicted embodiments, the bone 306 is a tibia, but those having ordinary skill in the art would understand that the invention is equally applicable to bone plating of other bones. The "drill bit" alternatively may be termed "twist drill." The bone plate 310 has a plurality of holes 312. In use, the drill guide assembly 10 is placed in contact with one of the holes 312. In some embodiments, the drill guide tip 110 rests on the bone plate 310. In other embodiments, the drill guide tip 110 protrudes through the hole 312 and rests on the bone 306. Optionally, the handle 20 may be used to place the drill guide assembly in contact with the hole 312 of the bone plate 310. Once the drill guide tip 110 is engaged with the bone plate 310, the user may rotate the collar 30. However, if the user is to drill on-axis, rotation may not be required. The user connects the drill bit 300 to a drill (not shown). The user inserts the drill bit 300 into the second bore 84. Optionally, the user may angle the drill bit 300 into the slot 42 and the cone 130. The outrigger 40 and the slot 42 provide support and alignment to a portion of the drill bit while drilling off-axis.

As best seen in FIG. 9, the drill bit may be placed on-axis (i.e., coaxial with the second bore 84), at the maximum angle at the end of the slot 42, or any place in between. In some embodiments, the second ball plunger 50 indicates the position from which the drill bit 300 moves from on-axis to off-axis. Thereafter, the user rotates the drill bit 300 using the drill to create a fastener hole in the bone 306. Optionally, the user may move the drill guide assembly 10 to another hole 312 and repeat the process.

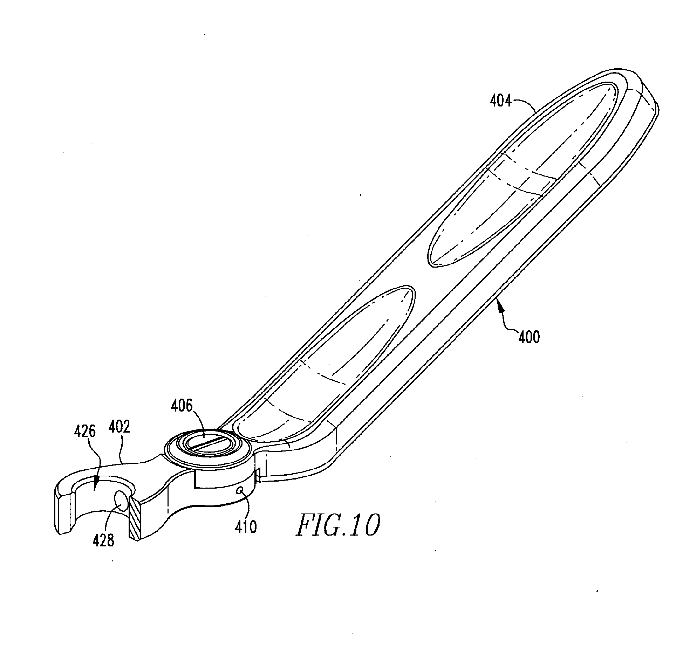

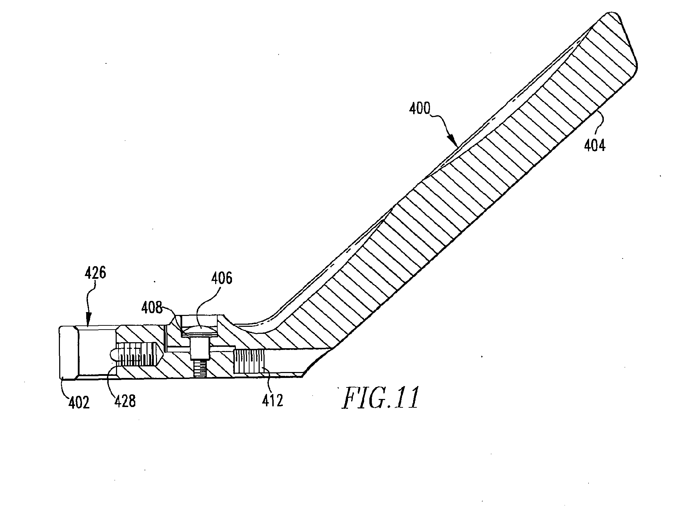

FIGS. 10 and 11 illustrate an alternative embodiment of the handle 400. The handle 400 includes a front pivot 402 and a grip portion 404. The pivoting handle 400 delivers enhanced accessibility and visibility through its multi-positional front pivot 402. The front portion 402 is connected to the grip portion 404 through the use of a fastener 406. In the depicted embodiment, the fastener 406 is a shoulder bolt. In some embodiments, the handle 400 also includes a disc spring or Belleville washer 408. The disc spring 408 places a biasing force on the fastener 406. The fastener 406 connects the grip portion 404 and the front pivot 402 in such a way that the front pivot 402 is pivotable relative to the grip portion. In some embodiments, the front pivot 402 is selectively positionable relative to the grip portion 404. In these embodiments, the front pivot and/or the grip portion has locking features that temporarily lock the front pivot in the desired location. For example, the front pivot 402 and/or the grip portion may have circumferentially spaced detents 410 and third ball plungers 412 that engage the detents 410. The handle 400 further includes an opening 426 to receive the collar 30 and may include a fourth ball plunger 428. The multi-positional rotating front pivot 402 rotates outside of the longitudinal axis of the drill guide itself for increased accessibility to the fracture site.

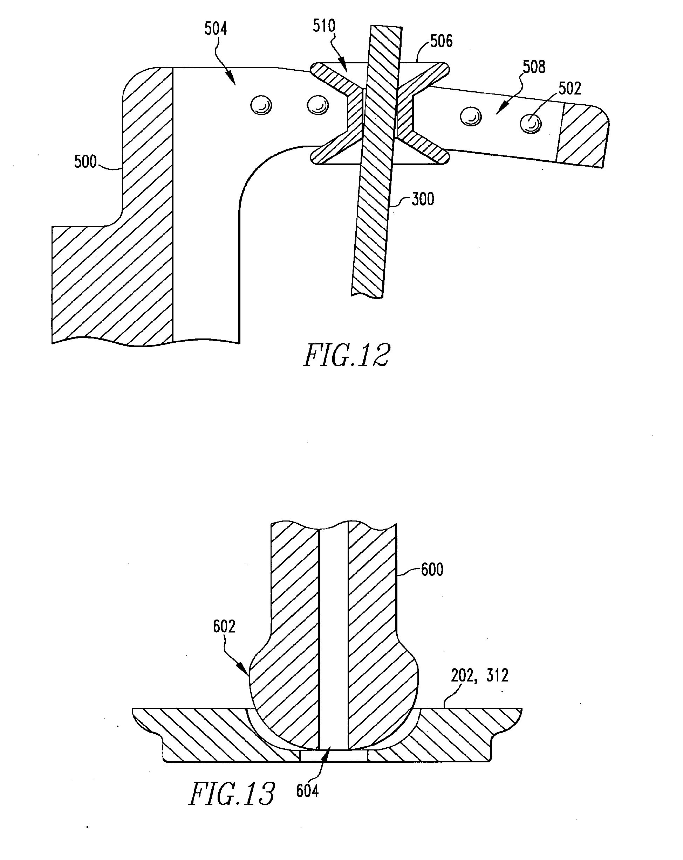

FIG. 12 illustrates an alternative embodiment of the outrigger 500. The outrigger 500 maintains alignment and supports the more proximal portion of the drill bit 300. The outrigger 500 may incorporate additional features that allow the user to positively identify off-axis drilling at a number of different angular degree increments. For example, the outrigger 500 may include detents or protrusions 502 located in a slot 504 and a flanged bobbin 506 that rides in the slot 504. The detents 502 are located such that spaces 508 positionally coincide with discrete angular position of the drill bit 300. The flanged bobbin 506 and the detents 502 are sized and dimensioned such that the flanged bobbin 506 can be pushed past the detents 502 but the detents 502 also temporarily hold the flanged bobbin 506 in one of the spaces 508. In some embodiments, the flanged bobbin 506 is constructed of a material, such as spring steel, that at least partially collapses when pressed against the detents 502 but returns to its normal size and shape when located in one of the spaces 508. The drill bit 300 may be inserted into an aperture 510 of the flanged bobbin 506, and the flanged bobbin 506 may be selectively positioned in the spaces 508 to angularly position the drill bit 300.

FIG. 13 illustrates an alternative embodiment of the guide sleeve 600. The guide sleeve 600 has a distal end portion 602 that has a ball-nose or spherical shape. The spherical shape allows the guide sleeve to swivel in the hole 202, 312 of the bone plate 200, 310. The guide sleeve 600 includes a second bore 604 that receives the drill bit 300.

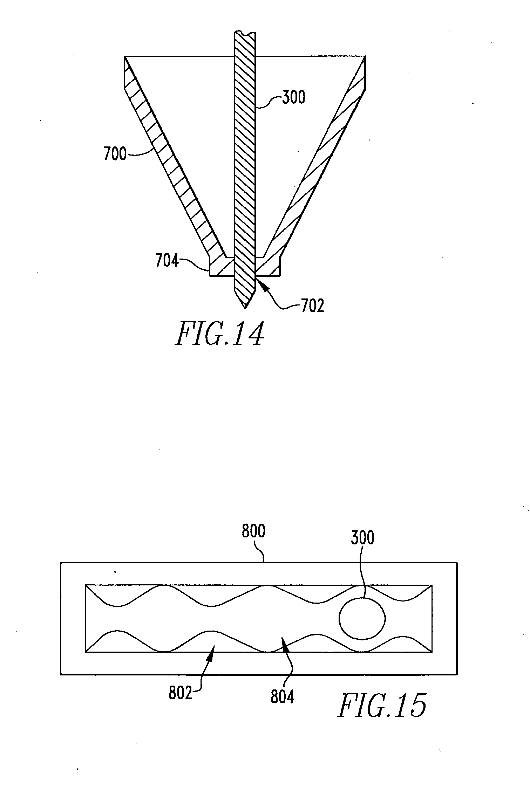

FIG. 14 illustrates another alternative embodiment of the drill sleeve 700. In this embodiment, a substantial portion of the drill sleeve 700 is in the shape of a cone that allows rotation about a central point. A user would define his or her own desired drilling angle, but limited by a maximum angle provided by the cone. The drill sleeve 700 includes a lower aperture 702 and may include an engagement portion 704 that engages with the bone plate 200, 310. The guide collar is not shown in the depicted embodiment.

FIG. 15 illustrates yet another embodiment of the outrigger 800. The outrigger 800 includes a series of scallops 802 that form spaces 804. The spaces 804 positionally coincide with discrete angular position of the drill bit 300. The scallops 802 and the spaces 804 are dimensioned such that the drill bit 300 easily rotates in the spaces 804 but slightly interferes with the scallops 802. In this manner, the drill bit 300 can be pushed past the scallops 802 but the scallops 802 also temporarily hold the drill bit 300 in one of the spaces 804. The drill bit 300 may be selectively positioned in one of the spaces 804 to select the angular position of the drill bit 300.

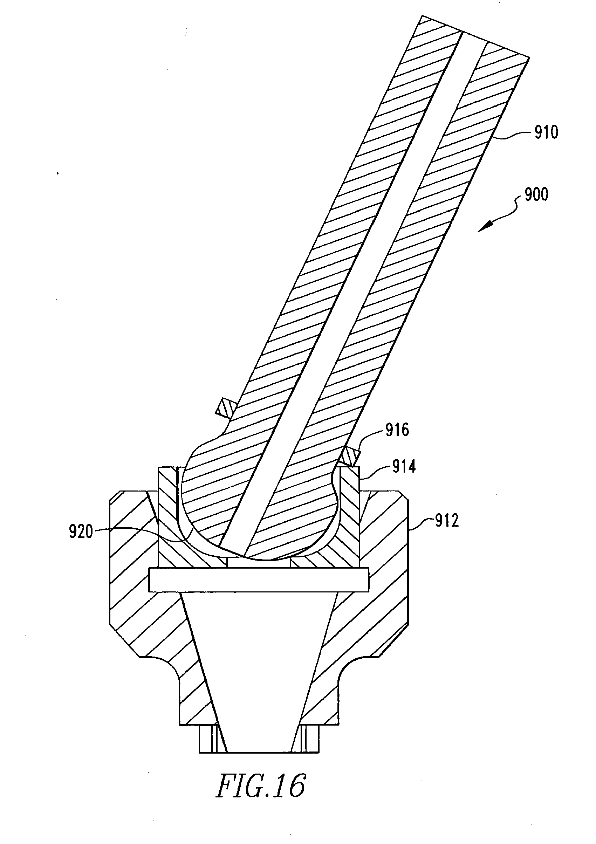

FIG. 16 illustrates the guide sleeve in a fourth embodiment. In this embodiment, there is a sleeve and tip assembly 900. The assembly 900 includes a guide sleeve 910, a distal guide tip 912, and a bushing 914. The guide sleeve 910 has a spherical tip 920 that mates with a corresponding spherical surface of the bushing 920. The combination of the spherical tip 920 and the bushing 920 allow the guide sleeve 910 to pivot and swivel relative to the distal guide tip 912. The resultant combination is similar to a ball joint rod bearing or a swivel bearing. In some embodiments, the bushing 914 is press fit into the distal guide tip 912, and the tightness of the press fit may be selected to achieve a desired amount of friction between the spherical tip 920 and the bushing 920. The amount friction may be selected such that the guide sleeve will temporarily hold a certain angle or allow for a desirable feel by the user.

The guide sleeve 910, the distal guide tip 912, and the bushing 914 each have a central opening to receive the drill bit 300 and also allow the drill bit to be positioned at a surgeon-selected angle relative to the bone plate through movement of the guide sleeve 910. The opening of the distal guide tip 912 may be a frusto-conical as shown, or the opening may have another shape, such as cylindrical.

In some embodiments, the assembly 900 further includes a limiter 916 connected to the guide sleeve 910. In the depicted embodiment, the limiter 916 is a washer affixed to the guide sleeve 910, such as by welding, but other structure may be used. What is significant is that the limiter 916 limits the relative angular position of the guide sleeve 910. The limiter 916 is placed on the guide sleeve 910 in such a way that the limiter 916 contacts a portion of the busing 920 to limit the maximum amount the guide sleeve 910 can pivot.

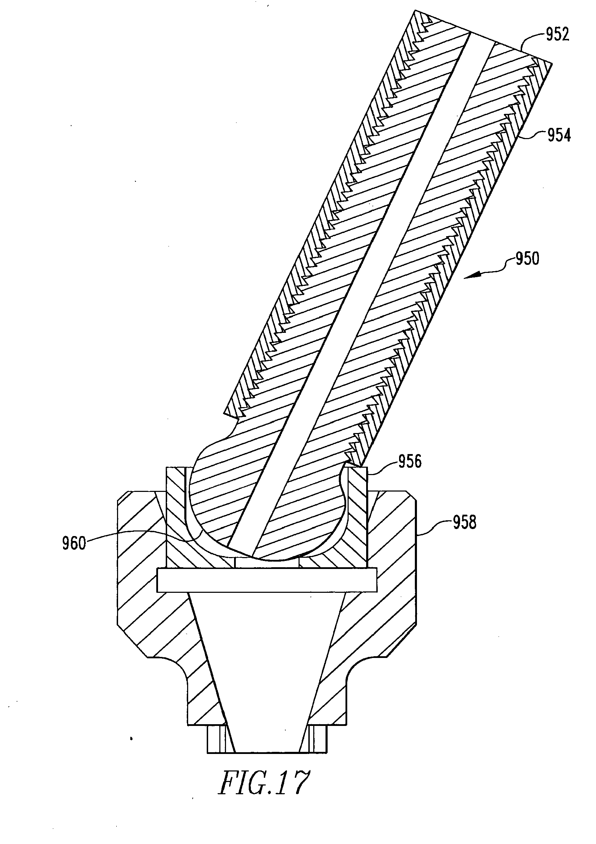

FIG. 17 illustrates the guide sleeve in a fifth embodiment. In this embodiment, there is a sleeve and tip assembly 950. The assembly 950 includes a guide sleeve 952, a bushing 956, a distal guide tip 958, and a limiter 954. The guide sleeve 952 has a spherical tip 960 that mates with the bushing 956. The combination of the spherical tip 960 and the bushing 956 allow the guide sleeve 952 to pivot and swivel relative to the distal guide tip 958. The guide sleeve 952, the distal guide tip 958, and the bushing 956 each have a central opening to receive the drill bit 300 and also allow the drill bit to be positioned at a surgeon-selected angle relative to the bone plate through movement of the guide sleeve 952. The opening of the distal guide tip 958 may be a frusto-conical as shown, or the opening may have another shape, such as cylindrical. The limiter 954 is threadingly engaged with the guide sleeve 952 and adjusted to contact a portion of the bushing 956 such that the limiter 954 limits the relative angular position of the guide sleeve 952. The limiter 954 may include one or more markings to indicate the selected maximum angle of the guide sleeve 952.

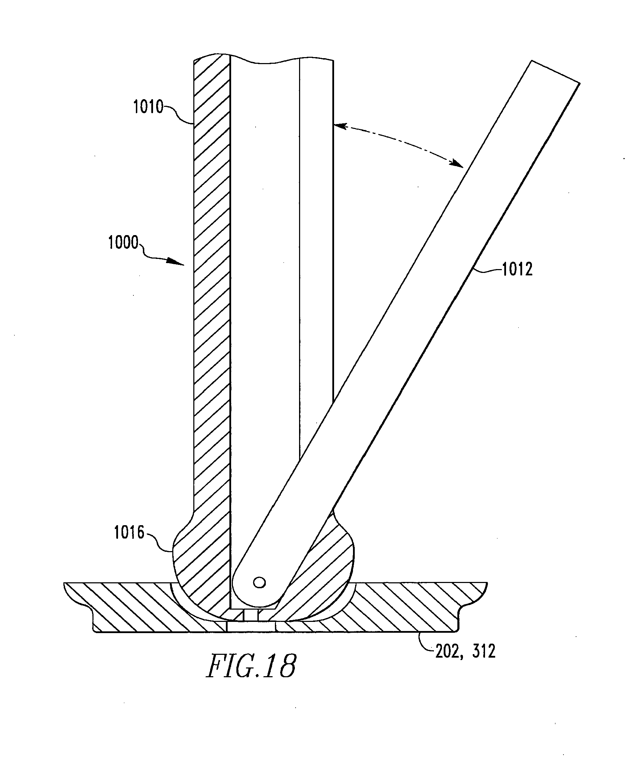

FIG. 18 illustrates the guide sleeve in a sixth embodiment. FIG. 18 illustrates a guide sleeve assembly 1000. The guide sleeve assembly 1000 includes an outer guide sleeve 1010 and an inner guide sleeve 1012. The inner guide sleeve 1012 is pivotably connected to the outer guide sleeve 1010 and includes an opening (not shown) to receive the drill bit 300. In the depicted embodiment, the outer guide sleeve 1012 has a spherical tip 1016 that engages the bone plate 202, 312. In other embodiments, however, the guide sleeve assembly 1000 may be used in connection with a distal guide tip 110, 912, 958. The outer guide sleeve 1010 allows the surgeon to swivel the inner guide sleeve 1012 to a desired position, and the inner guide sleeve 1012 pivots to allow the surgeon to select a desired drill angle. In some embodiments, the outer guide sleeve may limit the maximum angle the inner guide sleeve may pivot.

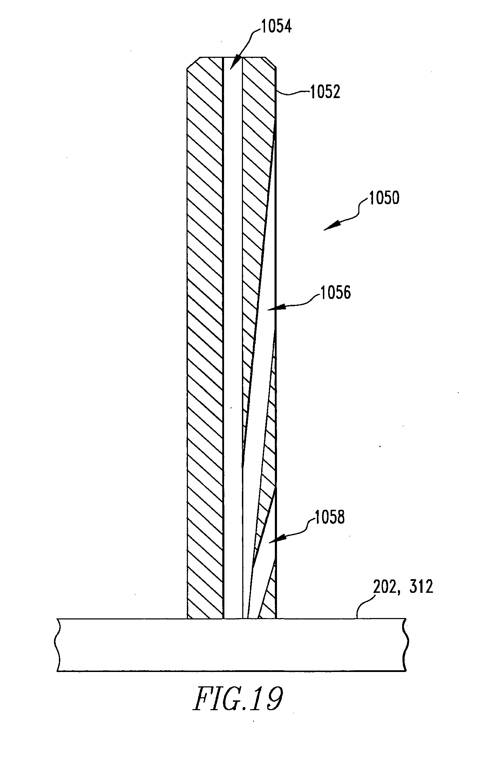

FIG. 19 illustrates the guide sleeve in a seventh embodiment. The guide sleeve of the seventh embodiment is similar to the guide sleeves 80 except the passage 90 is replaced by a plurality of holes. FIG. 19 illustrates a guide sleeve 1050. The guide sleeve 1050 has a wall 1052 and two or more openings constructed and dimensioned to receive the drill bit 300. The guide sleeve 1050 may be any shape but is cylindrical in the depicted embodiment. In the embodiment in FIG. 19, the guide sleeve 1050 has a first opening 1054, a second opening 1056, and a third opening 1058. In the depicted embodiment, the first opening 1054 is generally coaxial with the cylinder wall 1052, the second opening 1056 is about five degrees off-axis from the first opening 1054, and the third opening 1058 is about ten degrees off-axis from the first opening 1054. Those skilled in the art, however, would understand that any number of opening locations and angles of the openings may be used. Although not depicted, the guide sleeve 1050 may be used in conjunction with a spherical tip or a distal guide tip. Further, the guide sleeve 1050 may be used in conjunction with the guide collar.

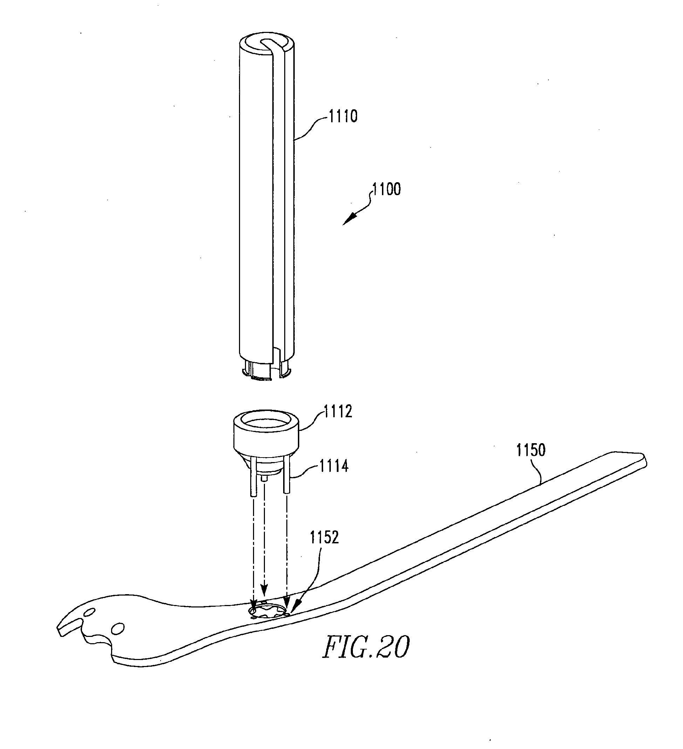

FIG. 20 illustrates an alternative embodiment of the drill guide assembly. FIG. 20 illustrates the drill guide assembly 1100. The drill guide assembly 1100 includes a drill sleeve 1110 and a drill guide tip 1112. In some embodiments, the drill guide assembly 1100 further includes a guide collar. The drill guide tip 1112 includes a plurality of locating members 1114. The locating members 1114 may be any number of shapes but are cylindrical in the depicted embodiment. In FIG. 20, three locating members 1114 are shown but any number of locating members may be used. The locating members 1114 correspond with and are adapted to engage locating holes 1152 located on bone plate 1150. In this embodiment, the guide sleeve 1110 is rotatable relative to the distal guide tip 1112. Unlike the embodiments depicted in FIGS. 4-7, the distal guide tip 1112 does not have a locating but instead utilizes the locating members 1114 to position the distal guide tip 1112 relative to the bone plate 1150. In some embodiments, the bone plate 1150 has sufficient thickness to allow the distal guide tip 1112 to pivot relative to the bone plate 1150.

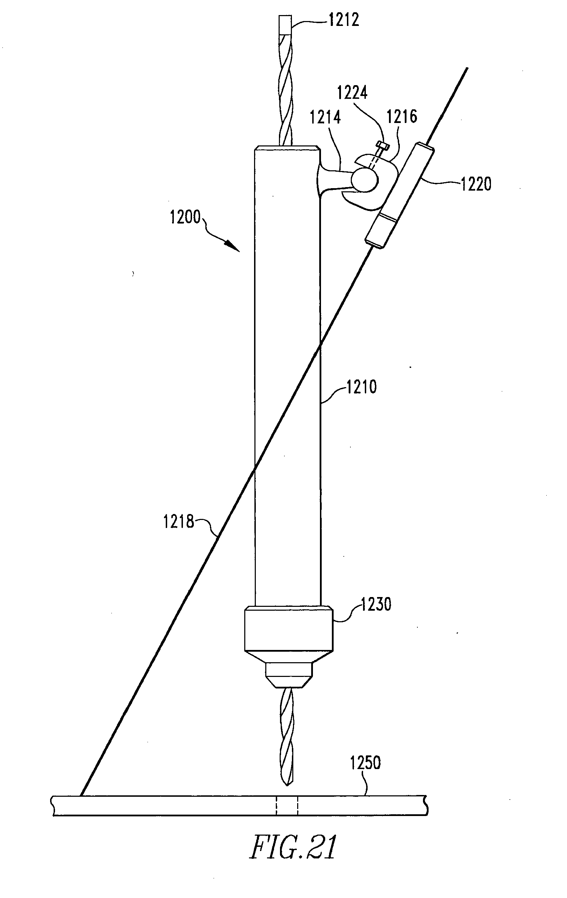

FIG. 21 is a front view of an alternative method of limiting the relationship between the bone plate and the drill guide assembly. FIG. 21 illustrates a drill guide assembly 1200 and a bone plate 1250. The drill guide assembly 1200 includes a drill sleeve 1210 and a drill guide tip 1230. The bone plate 1250 includes one or more holes (not shown). The drill guide assembly 1200 fits into the intended bone plate hole. The bone plate hole does not have a feature to hold the drill guide assembly 1200 at a particular angle or particular angular rotation relative to the hole. Therefore, the distal guide tip 1230 does not have a locating boss or locating member. The drill sleeve 1210 includes one or more spherical joints 1214. A wire holder 1220 operatively connects to the spherical joint 1214. In the depicted embodiment, a C-clamp and set screw 1224 are used to grip the spherical joint 1214. The wire holder 1220 is used to grip a wire 1218, such as a Kirschner wire, and apply a tensioning force to the drill guide assembly 1200 via the spherical joint 1214. As examples, the wire 1218 may be temporarily affixed to bone or the bone plate 1250, Several wire holders 1220 and wires 1218 may be necessary to adequately position and stabilize the drill guide assembly 1200. Thus, the surgeon may select a desired angular position and rotation for the drill guide assembly 1200 and use the anchored wires 1218 to temporarily affix the drill guide assembly in the chosen placement.

The invention also includes a method of drilling a hole into bone through a bone plate. The method includes the steps of engaging a guide tip of a variable angle drill guide assembly in contact with the bone plate, selecting a rotational position, inserting a drill bit into the variable angle drill guide assembly, selecting an angular position of the drill bit, and drilling the bone. Optional steps may include identifying the fracture type, selecting a bone plate, reducing the fracture, opening a surgical site, identifying an appropriate placement of the bone plate, temporarily affixing the bone plate into a desired position, and fastening the bone plate to the bone. The step of identifying the fracture type may include the step of x-raying the bone. The step of fracture reduction may be accomplished through manual reduction or through compression screws that engage the bone plate and provide axial compression. The step of temporarily affixing the bone plate may include the steps of clamping and/or attaching the bone plate via provisional fixation. The step of fastening the bone plate to the bone may be accomplished through the step of installing a locking screw and/or a non-locking screw.

In view of the foregoing, it will be seen that the several advantages of the invention are achieved and attained.

The embodiments were chosen and described in order to best explain the principles of the invention and its practical application to thereby enable others skilled in the art to best utilize the invention in various embodiments and with various modifications as are suited to the particular use contemplated.

As various modifications could be made in the constructions and methods herein described and illustrated without departing from the scope of the invention, it is intended that all matter contained in the foregoing description or shown in the accompanying drawings shall be interpreted as illustrative rather than limiting. For example, while FIG. 1 illustrates a distal guide tip, other structure and/or methods may be used to temporarily maintain contact between the guide sleeve and the bone plate. Thus, the breadth and scope of the present invention should not be limited by any of the above-described exemplary embodiments but should be defined only in accordance with the following claims appended hereto and their equivalents.

## Claims

1. A drill guide assembly adapted for removable connection to a bone plate and for guiding a drill bit through at least one hole of the bone plate, The at least one hole having a central axis, the drill guide assembly comprising:
a. a guide sleeve (600, 900, 952, 1000) having a sleeve body, a proximal end portion and a distal end portion, and a bore (84) extending through the sleeve body, wherein the distal end portion of the guide sleeve comprises a spherical tip and is adapted to swivel about the central axis of the at least one hole of the bone plate while the distal end portion maintains contact with the bone plate; and
b. a guide collar operatively connected to the proximal end portion of the guide sleeve.

2. The drill guide assembly according to claim 1, further comprising at least one ball plunger.

3. The drill guide assembly according to any claim 1 or claim 2, wherein the guide collar includes a base and a body portion.

4. The drill guide assembly according to any one of claims 1-3, wherein the guide sleeve further comprises at least one marker groove.

5. The drill guide assembly according to any one of claims 1-4, further comprising a drill guide handle, the drill guide handle having a grip portion and an engagement portion.

6. The drill guide assembly of claim 5, wherein the drill guide handle further comprises a pivot portion.

7. The drill guide assembly according to any one of claims wherein the guide sleeve comprises an outer guide sleeve (1010) and an inner guide sleeve (1012), wherein the inner guide sleeve is pivotably connected to the outer guide sleeve (1012) and includes an opening for receiving a drill bit.

8. The drill guide assembly according to any of claims 1-7, further comprising a bushing and a distal guide tip.

9. The drill guide assembly according to claim 7 or claim 8, further comprising a limiter.

10. The drill guide assembly according to claim 9, wherein the limiter threadingly engages the guide sleeve.

11. The drill guide assembly according to any one of claims 1-10, wherein the distal end portion of the guide sleeve comprises a drill guide tip.

12. The drill guide assembly according to claim 11, wherein the drill guide tip comprises an anti-rotation feature.

13. The drill guide assembly according to claim 12, wherein the anti-rotation feature is a locating boss.

14. The drill guide assembly according to claim 12, wherein the anti-rotation feature comprises at least one thread adapted to mate with at least one thread of the at least one bone plate hole.

15. The drill guide assembly of claim 12, wherein the distal guide tip includes a locating member.

16. The drill guide assembly according to any one of claims 10-15, wherein the drill guide tip further comprises a cone.
